# EUROPEAN PATENT APPLICATION

(11) **EP 1 849 500 A1**
(43) Date of publication of application: **31.10.2007**
(21) Application number: 06113129.8
(22) Date of filing: 26.04.2006
(51) Int. Cl.: A61Q 5/10, C07D 213/73, C07D 213/74, C07D 213/69, C07D 213/82, C07D 213/85

(54) **2, 3, 4, 6-Substituted pyridines and their use as hair dyeing compositions**

(71) Applicant: DyStar Textilfarben GmbH & Co. Deutschland KG, 65926 Frankfurt am Main (DE); Kao Corporation, Tokyo 131-8501 (JP)
(72) Inventor: Barbieru, Roxana, 65929 Frankfurt am Main (DE); Kühlwein, Jürgen, 63150 Heusenstamm (DE); Russ, Werner, 65439 Flörsheim-Wicker (DE); Pratt, Dominic, 64572 Büttelborn (DE); Möhring, Hartmut, 64342 Seeheim-Jugenheim (DE)
(74) Representative: Muley, Ralf

(57) **Abstract**

The present invention refers to hair dye compositions comprising a compound of formula (I) 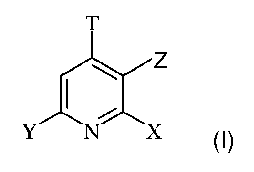 wherein T, Z, X and Y are defined as given in claim 1.

## Description

The present invention relates to novel hair dye composition comprising substituted pyridine compounds as coupling compounds.

It is known to colour human hair with dyeing compositions containing oxidation dye precursors, also called oxidation bases or developers.
Oxidation bases are colourless or weakly coloured compounds, such as ortho- or para-phenylenediamines, ortho- or para-aminophenols or heterocyclic compounds and react with oxidizing agents to give coloured compounds.
The obtained shades can be varied by combining the oxidation bases with couplers, such as aromatic meta-diamines, meta-aminophenols, meta-hydroxyphenols and certain heterocyclic compounds. A variety of oxidation bases and couplers can be used to enable to get a broad range of different shades.
The permanent colours obtained by using these oxidation dyes need to fulfil some requirements. They should be safe, provide good intensity evenly along the hair shaft, should be stable to external influences, such as shampooing, light, sweat and rubbing and should enable the coverage of grey hair. The dyes should also be stable in the formulation.
There is a constant need to get new developers and couplers that improve at least one of these requirements, particularly there is a constant need to find new couplers which provide improved colour properties

Substituted pyridine compounds are known and for example used as coupling components in- the preparation of azo dyestuffs, see for example DE 2062717, DE 2230392 and DE 2515662. The use of such compounds as coupling compounds to produce oxidation dyes for hair dyeing is also known, see for example EP 0 063 736 A2, EP 1,312,606 A1, EP 1,372,585 A1, EP 1, 413,286 A1, EP 1,488,783 A1, EP 1,551,806 A1, WO 2001 /00142 and DE 100 30 910.

It was now surprisingly found that specific pyridine compounds according to the definition given below can be used as couplers and fulfil the needs mentioned above.

The present invention refers to hair dye compositions comprising at least one compound of formula (I) 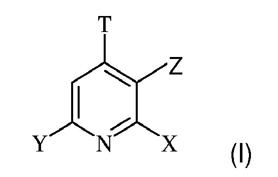 wherein
each of X and Y, independently, are -NR¹R², -NHSO₂R³, -NHC(O)R⁴or -OR⁵;
Z is alkyl, -CH₂NR⁶R⁷, -CH₂N(SO₂R⁸)R⁹, -CH₂N(C(O)R¹⁰)R¹¹, -CH₂OR¹², -C(O)NR¹³R¹⁴, -C(S)NR¹⁵R¹⁶, -C(O)OR¹⁷, -CH=NR¹⁸, -C(alkoxy)=NR¹⁹, -C(O)R²⁰ or - C(S)R²¹;
T is alkyl, -OR²², -SR²³, or -C(O)OR²⁴;
each of R¹ to R⁵, independently, are alkyl, hydroxyalkyl, polyhydroxylalkyl, arylalkyl, alkoxyalkyl, thioalkoxyalkyl, poly(oxyalkylene)alkyl, aminoalkyl, N-monoalkyl-aminoalkyl, N-monoaryl-aminoalkyl, N,N-dialkyl-aminoalkyl, N,N-diaryl-aminoalkyl, N-alkyl-N-aryl-aminoalkyl, amino-hydroxy-alkyl, alkoxyalkylaminoalkyl, thioalkoxyalkyl-aminoalkyl, aminoalkyloxyalkyl, N-monoalkylamino-alkyloxyalkyl, N,N-dialkyl-aminoalkoxyalkyl, N-arylamino-alkoxyalkyl, N,N-diarylaminoalkoxyalkyl, N-alkyl-N-aryl-amino-alkoxyalkyl, aminoalkylthioxyalkyl, N-monoalkylamino-alkylthioxy-alkyl, N,N-dialkylamino-alkylthioxyalkyl, N-arylamino-alkylthioxyalkyl, N,N-diarylaminoalkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl; and
R¹, R² and R⁵ can additionally be hydrogen; or
R¹ and R² form together with the nitrogen to which they are bonded a 5- or 6-membered heterocyclic ring; and
each of R⁶to R²¹, independently are, alkyl, hydroxyalkyl, polyhydroxylalkyl, arylalkyl, alkoxyalkyl, thioalkoxyalkyl, poly(oxyalkylene)alkyl, aminoalkyl, N-monoalkyl-aminoalkyl, N-monoaryl-aminoalkyl, N;N-dialkyl-aminoalkyl, N;N-diaryl-aminoalkyl, N-alkyl-N-aryl-aminoalkyl, amino-hydroxy-alkyl, alkoxyalkylaminoalkyl, thioalkoxyalkyl-aminoalkyl, aminoalkyloxyalkyl, N-monoalkylamino-alkoxyalkyl, N,N-dialkylamino-alkoxyalkyl, N-arylamino-alkoxyalkyl, N,N-diarylaminoalkoxyalkyl, N-alkyl-N-aryl-amino-alkoxyalkyl, aminoalkylthioxyalkyl, N-monoalkylamino-alkylthioxyalkyl, N,N-dialkylaminoalkylthioxyalkyl, N-arylamino-alkylthioxyalkyl, N,N-diarylamino-alkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl; and
R⁶, R⁷, R⁹ and R¹¹ to R²⁰ can additionally be hydrogen;
each of R²² to R²⁴, independently are hydrogen, alkyl, hydroxyalkyl, polyhydroxylalkyl, arylalkyl, alkoxyalkyl, thioalkoalkyl, poly(oxyalkylene)alkyl, aminoalkyl, N-monoalkyl-aminoalkyl, N-monoaryl-aminoalkyl, N,N-dialkyl-aminoalkyl, N,N-diaryl-aminoalkyl, N-alkyl-N-aryl-aminoalkyl, amino-hydroxy-alkyl, alkoxyalkylaminoalkyl, thioalkoxyalkylaminoalkyl, aminoalkyloxyalkyl, N-monoalkylamino-alkyloxyalkyl, N,N-dialkylaminoalkyloxyalkyl, N-arylamino-alkyloxyalkyl, N,N-diarylaminoalkyloxyalkyl, N-alkyl-N-aryl-amino-alkyloxyalkyl, aminoalkylthioxyalkyl, N-monoalkylamino-alkyl-thioxyalkyl, N,N-dialkylaminoalkyl-thioxyalkyl, N-arylamino-alkylthioxyalkyl, N,N-diarylaminoalkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl; whereby
Z and T cannot be both alkyl;
when X and Y both are -OR⁵ and R⁵ is hydrogen, then Z cannot be alkyl or -CH₂OR¹² with R¹² being hydrogen; and
when Z is -C(O)NR¹³R¹⁴ with R¹³ and R¹⁴ both being hydrogen or one of R¹³ and R¹⁴ being hydrogen and the other methyl, then X and Y cannot both be -NR¹R² with R¹ and R² being hydrogen.

Alkyl groups may be straight-chain or branched and are preferably (C₁-C₆)-alkyl groups, for example methyl, ethyl, n-propyl, isopropyl, n-butyl, n-pentyl, isopentyl or n-hexyl. The same logic applies for alkoxy groups which accordingly are preferably (C₁-C₆)-alkoxy, for example methoxy and ethoxy.
Thioalkoxy groups are preferably (C₁-C₆)-thioalkoxy, for example -SCH₃ or -SC₂H₅.
Cycloalkyl is preferably (C₃-C₈)-cycloalkyl and especially preferably cyclopentyl and cyclohexyl.

Aryl groups are preferably phenyl or naphthyl groups.
A 5- or 6- membered heterocyclic ring which is formed by R¹ and R² is preferably a pyrrolidine, piperidine, morpholine or piperazine ring.
Heteroaryl groups are preferably pyridine, pyrimidine, pyridazine, pyrazine, pyrrole, imidazole, pyrazole, 1,2,4-triazole, tetrazole, thiophene, thiazole, isothiazole, 1,3,4-thiadiazole, furane, oxazole and isoxazole.
Heterocycloalkyl groups are preferably pyrrolidine, piperidine, morpholine and piperazine.

Preferred hair dye compositions according to the present invention comprise compounds of the formula (Ia) 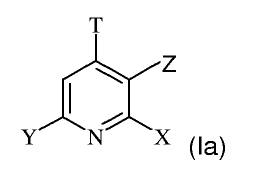 wherein
each of X and Y, independently, are -NR¹R²;
Z is -CH₂NR⁶R⁷, -CH₂N(SO₂R⁸)R⁹, -CH₂N(C(O)R¹⁰)R¹¹, -CH₂OR¹², -C(O)NR¹³R¹⁴or -C(S)NR¹⁵R¹⁶;
T is (C₁-C₆)-alkyl;
each of R¹, R² and R⁶to R¹⁶, independently, are (C₁-C₆)-alkyl, hydroxy-(C₁-C₆)-alkyl, amino-(C₁-C₆)-alkyl, N-mono-(C₁-C₆)-alkyl-amino-(C₁-C₆)-alkyl or N,N-di-(C₁-C₆)-alkyl-amino-(C₁-C₆)-alkyl and
R¹, R², R⁶, R⁷, R⁹ and R¹¹ to R¹⁶ can additionally be hydrogen,
whereby the compounds wherein Z is -CONH₂ or -CONHCH₃ and X and Y are both - NH₂ are excluded.
Especially preferred hair dye compositions comprise compounds of the formula (la) wherein
each of X and Y, independently, are -NR¹R²;
Z is aminomethyl or -CONH₂;
T is methyl or ethyl;
R¹ is hydrogen; and
R² is hydrogen, methyl, ethyl, hydroxymethyl, hydroxyethyl, dimethylaminomethyl, diethylaminomethyl, dimethylaminoethyl or diethylaminoethyl,
whereby the compounds wherein Z is -CONH₂ and X and Y are both -NH₂ are excluded.

Examples of compounds of the formula (la) are the compounds of the formulae (ld) to (In)

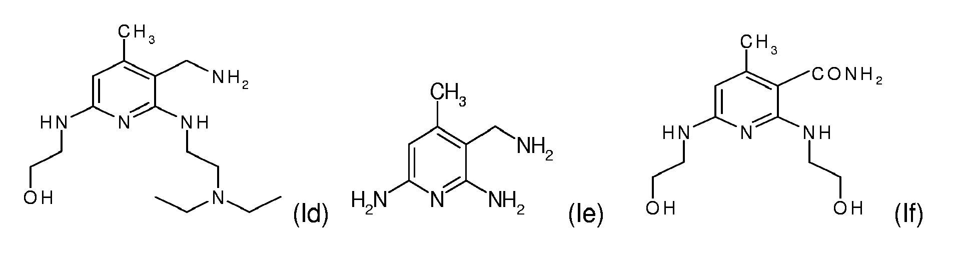

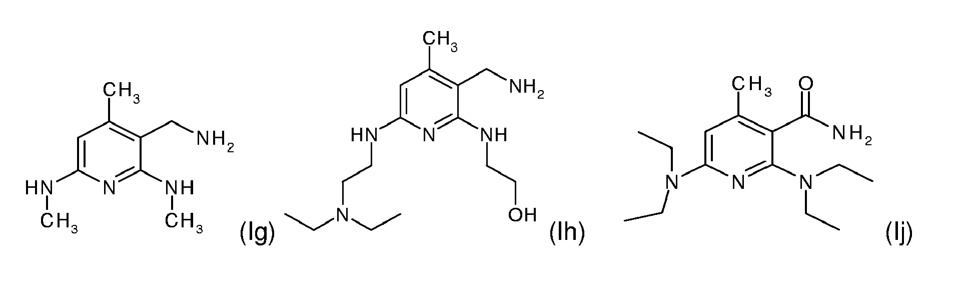

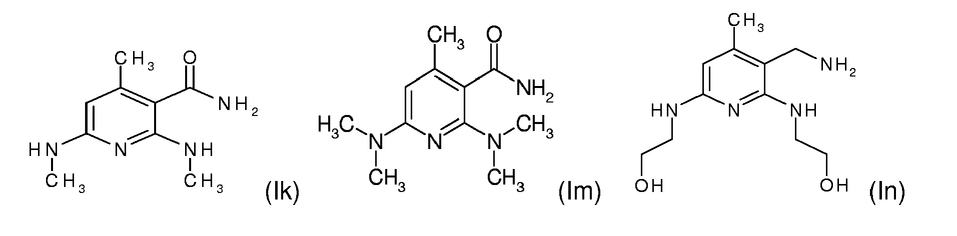

Further preferred hair dye compositions comprise compounds of the formula (Ib) 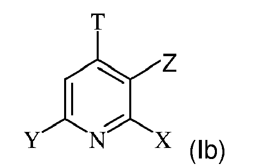 wherein
each of X and Y, independently, are -OR⁵;
Z is -C(O)NR¹³R¹⁴ or -C(O)OR¹⁷;
T is (C₁-C₆)-alkyl, -OR²² or -C(O)OR²⁴; and
each of R⁵, R¹³, R¹⁴, R¹⁷, R²² and R²⁴, independently, are hydrogen or (C₁-C₆)-alkyl.

Especially preferred hair dye compositions comprise compounds of the formula (lb) wherein
X and Y are -OH;
Z is -CONH₂, -COOCH₃ or -COOC₂H₅; and
T is hydroxy, methyl or ethyl, -COOCH₃ or -COOC₂H₅.

Examples of compounds of the formula (Ib) are the compounds of the formulae (Io) to (Iq)

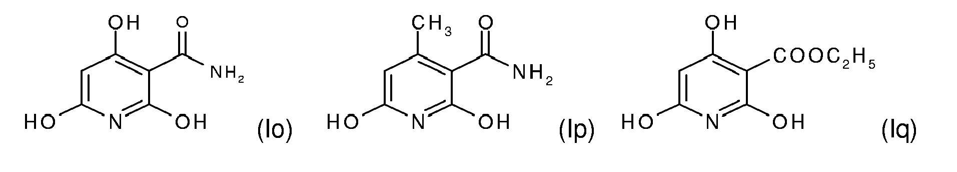

Still further preferred hair dye compositions comprise compounds of the formula (Ic) 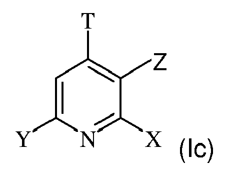 wherein
one of X and Y is -NR¹R² and the other is -OR⁵;
Z is -C(O)NR¹³R¹⁴;
T is (C₁-C₆)-alkyl; and
each of R¹, R², R⁵, R¹³ and R¹⁴, independently, are hydrogen or (C₁-C₆)-alkyl.

Examples of compounds of the formula (lc) are the compounds of the formulae (Ir) to (Iu)

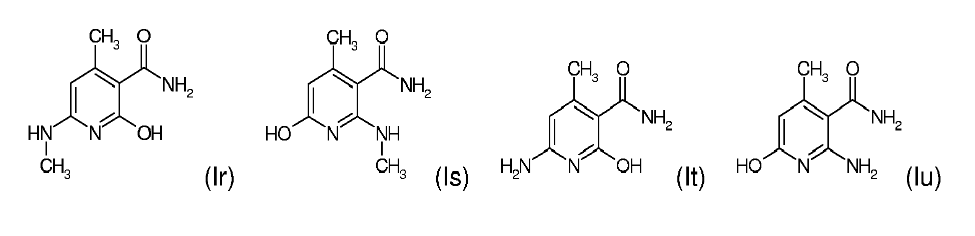

The compounds of the formula (I) can be present in the hair dye compositions according to the present invention in form of their acid addition salts of organic or inorganic acids. Examples of such acid additions salts are chlorides, sulfates, phosphates, acetates, propionates, lactates and citrates.

The inventive hair dye compositions preferably comprise in addition to the compounds of the formula (I) as couplers one or more developers, which couplers and developers are capable to form an oxidation dye for hair dyeing.

Preferred developers are p-phenylenediamine derivatives such as benzene-1,4-diamine (commonly known as p-phenylenediamine), 2-methyl-benzene-1,4-diamine, 2-chloro-benzene-1,4-diamine, N-phenyl-benzene-1,4-diamine, N-(2-ethoxyethyl)benzene-1,4-diamine, 2-[(4-amino-phenyl)-(2-hydroxy-ethyl)-amino]-ethanol (commonly known as N,N-bis(2-hydroxyethyl)-p-phenylenediamine) (2,5-diamino-phenyl)-methanol, 1-(2,5-diamino-phenyl)-ethanol, 2-(2,5-diamino-phenyl)-ethanol, N-(4-aminophenyl)benzene-1,4-diamine, 2,6-dimethyl-benzene-1,4-diamine, 2-isopropyl-benzene-1,4-diamine, 1-[(4-aminophenyl)amino]-propan-2-ol, 2-propylbenzene-1,4-diamine, 1,3-bis[(4-aminophenyl)(2-hydroxyethyl)-amino]propan-2-ol, N⁴,N⁴,2-trimethylbenzene-1,4-diamine, 2-methoxy-benzene-1,4-diamine, 1-(2,5-diaminophenyl)ethane-1,2-diol, 2,3-dimethyl-benzene-1,4-diamine, N-(4-amino-3-hydroxy-phenyl)-acetamide, 2,6-diethylbenzene-1 ,4-diamine, 2,5-dimethylbenzene-1,4-diamine, 2-thien-2-ylbenzene-1,4-diamine, 2-thien-3-ylbenzene-1,4-diamine, 2-pyridin-3-ylbenzene-1,4-diamine, 1,1'-biphenyl-2,5-diamine, 2-(methoxymethyl)benzene-1,4-diamine, 2-(aminomethyl)benzene-1,4-diamine, 2-(2,5-diaminophenoxy)ethanol, N-[2-(2,5-diaminophenoxy)ethyl]-acetamide, N,N-dimethylbenzene-1,4-diamine, N,N-diethylbenzene-1,4-diamine, N,N-dipropylbenzene-1,4-diamine, 2-[(4-aminophenyl)(ethyl)amino]ethanol, 2-[(4-amino-3-methyl-phenyl)-(2-hydroxy-ethyl)-amino]-ethanol, N-(2-methoxyethyl)-benzene-1,4-diamine, 3-[(4-aminophenyl)amino]propan-1-ol, 3-[(4-aminophenyl)-amino]propane-1,2-diol, N-{4-[(4-aminophenyl)amino]butyl{benzene-1,4-diamine, and 2-[2-(2-}-2-[(2,5-diaminophenyl)-oxy]ethoxy}ethoxy)ethoxy]benzene-1,4-diamine; p-aminophenol derivatives such as 4-amino-phenol (commonly known as p-aminophenol), 4-methylamino-phenol, 4-amino-3-methyl-phenol, 4-amino-2-hydroxymethyl-phenol, 4-amino-2-methyl-phenol, 4-amino-2-[(2-hydroxy-ethylamino)-methyl]-phenol, 4-amino-2-methoxymethyl-phenol, 5-amino-2-hydroxy-benzoic acid, 1-(5-amino-2-hydroxy-phenyl)-ethane-1 ,2-diol, 4-amino-2-(2-hydroxy-ethyl)-phenol, 4-amino-3-(hydroxymethyl)phenol, 4-amino-3-fluoro-phenol, 4-amino-2-(aminomethyl)-phenol, and 4-amino-2-fluoro-phenol; o-aminophenol derivatives such as 2-amino-phenol (commonly known as o-aminophenol), 2,4-diaminophenol, 2-amino-5-methyl-phenol, 2-amino-6-methylphenol, N-(4-amino-3-hydroxy-phenyl)-acetamide, and 2-amino-4-methyl-phenol; benzene-1,2-4-triol and heterocyclic derivatives such as pyrimidine-2,4,5,6-tetramine (commonly known as 2,4,5,6-tetraaminopyridine), 1-methyl-1H-pyrazole-4,5-diamine, 2-(4,5-diamino-1H-pyrazol-1-yl)ethanol, N<2> ,N<2> -dimethyl-pyridine-2,5-diamine, 2-[(3-amino-6-methoxypyridin-2-yl)amino]ethanol, 6-methoxy-N<2> -methyl-pyridine-2,3-diamine, 2,5,6-triaminopyrimidin-4(1H)-one, pyridine-2,5-diamine, 1-isopropyl-1H-pyrazole-4,5-diamine, 1-(4-methylbenzyl)-1H-pyrazole-4,5-diamine, 1-(benzyl)-1H-pyrazole-4,5-diamine, 1-(4-chlorobenzyl)-1H-pyrazole-4,5-diamine and 2,5,6-triamino-4-pyrimidinol sulfate.

The hair dye compositions according to the present invention can in addition to the compounds of the formula (I) comprise further couplers. Such further couplers are preferred couplers as normally used to produce oxidation dyes for hair dyeing. Examples of such couplers are phenols, resorcinol and naphthol derivatives such as naphthalene-1,7-diol, benzene-1,3-diol, 4-chlorobenzene-1,3-diol, naphthalen-1-ol, 2-methyl-naphthalen-1-ol, naphthalene-1,5-diol, naphthalene-2,7-diol, benzene-1,4-diol, 2-methyl-benzene-1,3-diol, 7-amino-4-hydroxy-naphthalene-2-sulfonic acid, 2-isopropyl-5-methylphenol, 1,2,3,4-tetrahydro-naphthalene-1,5-diol, 2-chlorobenzene-1,3-diol, 4-hydroxy-naphthalene-1-sulfonic acid, benzene-1,2,3-trio!, naphthalene-2,3-diol, 5-dichloro-2-methylbenzene-1,3-diol, 4,6-dichlorobenzene-1,3-diol, and 2,3-dihydroxy-[1,4]naphthoquinone;
m-phenylenediamines such as 2,4-diaminophenol, benzene-1,3-diamine, 2-(2,4-diamino-phenoxy)-ethanol, 2-[(3-amino-phenyl)-(2-hydroxy-ethyl)-amino]-ethanol, 2-mehyl-benzene-1,3-diamine, 2-[[2-(2,4-diamino-phenoxy)-ethyl]-(2-hydroxy-ethyl)-amino]-ethanol, 4-{3-[(2,4-diaminophenyl)oxy]propoxy}benzene-1,3-diamine, 2-(2,4-diamino-phenyl)-ethanol, 2-(3-amino-4-methoxy-phenylamino)-ethanol, 4-(2-amino-ethoxy)-benzene-1,3-diamine, (2,4-diamino-phenoxy)-acetic acid, 2-[2,4-diamino-5-(2-hydroxy-ethoxy)-phenoxy]-ethanol, 4-ethoxy-6-methyl-benzene-1,3-diamine, 2-(2,4-diamino-5-methyl-phenoxy)-ethanol, 4,6-dimethoxy-benzene-1,3-diamine, 2-[3-(2-hydroxy-ethylamino)-2-methyl-phenylamino]-ethanol, 3-(2,4-diamino-phenoxy)-propan-1-ol, N-[3-(dimethylamino)phenyl]urea, 4-methoxy-6-methylbenzene-1 ,3-diamine, 4-fluoro-6-methylbenzene-1,3-diamine, 2-({3-[(2-hydroxyethyl)amino]-4,6-dimethoxyphenyl}-amino)ethanol, 3-(2,4-diaminophenoxy)-propane-1,2-diol, 2-[2-amino4-(methylamino)-phenoxy]ethanol, 2-[(5-amino-2-ethoxy-phenyl)-(2-hydroxyethyl)-amino]-ethanol, 2-[(3-aminophenyl)amino]ethanol, N-(2-aminoethyl)benzene-1,3-diamine, 4{[(2,4-diamino-phenyl)oxy]methoxy}-benzene-1,3-diamine, and 2,4-dimethoxybenzene-1,3-diamine;
m-aminophenols such as 3-amino-phenol, 2-(3-hydroxy-4-methyl-phenylamino)-acetamide, 2-(3-hydroxy-phenylamino)-acetamide, 5-amino-2-methyl-phenol, 5-(2-hydroxy-ethylamino)-2-methyl-phenol, 5-amino-2,4-dichloro-phenol, 3-amino-2-methyl-phenol, 3-amino-2-chloro-6-methyl-phenol, 5-amino-2-(2-hydroxy-ethoxy)-phenol, 2-chloro-5-(2,2,2-trifluoro-ethylamino)-phenol, 5-amino-4-chloro-2-methylphenol, 3-cyclopentylamino-phenol, 5-[(2-hydroxyethyl)amino]4-methoxy-2-methylphenol, 5-amino-4-methoxy-2-methylphenol, 3-(dimethylamino)phenol, 3-(diethylamino)phenol, 5-amino4-fluoro-2-methylphenol, 5-amino-4-ethoxy-2-methylphenol, 3-amino-2,4-dichloro-phenol, 3-[(2-methoxyethyl)amino]phenol, 3-[(2-hydroxyethyl)amino]phenol, 5-amino-2-ethyl-phenol, 5-amino-2-methoxyphenol, 5-[(3-hydroxypropyl)amino]-2-methylphenol, 3-[(3-hydroxy-2-methylphenyl)-amino]propane-1,2-diol, and 3-[(2-hydroxyethyl)amino]-2-methylphenol; and
heterocyclic derivatives such as: 3,4-dihydro-2H-1,4-benzoxazin-6-ol, 4-methyl-2-phenyl-2,4-dihydro-3H-pyrazol-3-one, 6-methoxyquinolin-8-amine, 4-methylpyridine-2,6-diol, 2,3-dihydro-1,4-benzodioxin-5-ol, 1,3-benzodioxol-5-ol, 2-(1,3-benzodioxol-5-ylamino)ethanol, 3,4-dimethylpyridine-2,6-diol, 5-chloropyridine-2,3-diol, 2,6-dimethoxypyridine-3,5-diamine, 1,3-benzodioxol-5-amine, 2-{[3,5-diamino-6-(2-hydroxy-ethoxy)-pyridin-2-yl]oxy}-ethanol, 1H-indol-4-ol, 5-amino-2,6-dimethoxypyridin-3-ol, 1H-indole-5,6-diol, 1H-indol-7-ol, 1H-indol-5-ol, 1H-indol-6-ol, 6-bromo-1,3-benzodioxol-5-ol, 2-aminopyridin-3-ol, 3-[(3,5-diaminopyridin-2-yl)oxy]propane-1,2-diol, 5-[(3,5-diaminopyridin-2-yl)oxy]pentane-1,3-diol, 1H-indole-2,3-dione, indoline-5,6-diol, 3,5-dimethoxypyridine-2,6-diamine, 6-methoxypyridine-2,3-diamine, and 3,4-dihydro-2H-1,4-benzoxazin-6-amine.

The inventive hair dye compositions usually contain couplers at a total concentration of 0.001 to 10%, preferably 0.005 to 7.5% and more preferably 0.01 to 5% by weight, based on the weight of the hair dyeing composition and developers at a total concentration of 0.001 to 10%, preferably 0.005 to 7.5% and more preferably 0.01 to 5% by weight, calculated based on the weight of the hair dyeing composition.

The hair dye compositions according to the present invention can additionally comprise at least one direct dye. Such direct dyes can be cationic, anionic or neutral and can be of natural or synthetic origin. Preferred natural dyes are plant dyestuffs. Suitable dyes of such types are known on the market for hair colouring applications.

Suitable cationic dyestuffs are for example Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57, Basic Orange 31 and Basic Yellow 87. Additional examples are the dyes disclosed in WO 95/15144 which are included herein by reference.
The hair dye compositions according to the present invention can contain cationic dyestuffs at a concentration of 0.001 to 2%, preferably 0.005 to 1.5% and more preferably 0.01 to 1 % by weight, based on the weight of the hair dyeing composition.

Suitable anionic dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium and potassium.
The hair dye compositions according to the present invention can contain anionic dyestuffs at a concentration of 0.001 to 2%, preferably 0.005 to 1.5% and more preferably 0.01 to 1 % by weight, based on the weight of the hair dyeing composition.

Suitable neutral dyes (HC dyes or nitro dyes) are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.l3, HC Yellow No.l4, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

The hair dye compositions according to the present invention can contain neutral dyestuffs at a concentration of 0.001 to 2%, preferably 0.01 to 1.5% and more preferably 0.05 to 1 % by weight, based on the weight of the hair dyeing composition.

Suitable plant dyestuffs are henna (red or black), alkanna root, laccaic acid, indigo, logwood powder, madder root and rhubarb powder.
The pH of the hair dye compositions according to the present invention may range from 3 to 12, preferably from 5 to 11. The pH might be adjusted to the desirable value by the use of acidifying and alkalising agents.
Suitable acidifying agents are for example mineral and organic acids, such as hydrochloric acid, orthophosphoric acid, sulfuric acid, carboxylic acids such as acetic acid, tartaric acid, citric acid, and lactic acid, and sulphonic acids.
Suitable alkalizing agents are for example aqueous ammonia, alkali metal carbonates, alkalonamine such as monoethanolamine, diethanolamine, and triethanolamine, and derivatives thereof, sodium hydroxide.

In addition to the above-described components, those ordinarily employed as a raw material for cosmetics can be added to the hair dye compositions of the present invention. Examples of such an optional component include hydrocarbons such as squalane; animal or vegetable fats and oils; higher fatty acids such as oleic acid; organic solvents such as 1,2-propylene glycol; penetration promoters; cationic surfactants; natural or synthetic polymers; higher alcohols such as cetearyl alcohol; ethers; amphoteric surfactants; nonionic surfactants such as stearoyl monoethanolamide, coconut monoethanolamide, propylene glycol monostearate, polyoxyethylene (5) coconut amide, anionic surfactants such as sodium cetyl sulfate, sodium stearyl sulfate, potassium stearate, sodium stearate, polyoxyethylene (5) oleyl ether phosphate; protein derivatives; active ingredients such as coenzyme Q10; amino acids; antiseptics; chelating agents; stabilizers; antioxidants; plant extracts; crude drug extracts; vitamins such as retinyl palmitate; colorants; perfumes; catalysts such as potassium iodide; and ultraviolet absorbers.

The hair dye compositions of the present invention can be prepared in a conventional manner by mixing the components in the required amounts.

In order to use the hair dye compositions of the present invention to dye hair they have to be mixed with an oxidation agent, which allows the formation of an oxidation dye by reaction of the coupler component with the developer component and which is usually called developer composition.
Preferred oxidation agents are peroxide containing agents, particularily hydrogen peroxide, or precursors thereof. Also suitable are urea peroxide, sodium perborate, sodium percarbonate, melamine peroxide, and persulfates such as ammonium persulfate, sodium persulfate and potassium persulfate. Oxygen can also be used as oxidation agent.
Typically, hydrogen peroxide or its addition compounds with urea, melamine, sodium borate or sodium carbonate are used in the form of a 1 % to 20%, preferably 2% to 15 %, most preferably 2% to 12% preparation.

The hair dye composition according to the present invention as well as the oxidation agent can be present in the form of powder, transparent liquid, emulsion, cream, gel, paste, aerosol, aerosol foam or the like.
The present application also refers to a hair dyeing kit which comprises in separate containers a hair dye composition according to the present invention, a developer composition and optionally further components.
Such further components are for example a shampoo, a conditioning composition, a hair treatment product, gloves and instructions for use.

Usually, the hair dye composition according to the present invention and the developer composition (oxidation agent) are mixed just before hair dyeing and the mixture is applied to the hair in a sufficient amount, which depends on the hair abundance, generally from about 60 to 200 grams.
The mixture is then allowed to act on the hair for about 10 to about 45 minutes, preferably about 30 minutes, at about 15 to 50°C. Thereafter, the hair is rinsed with water and dried. If necessary, it is washed with a shampoo. Subsequently the hair is dried.

The hair dye composition according to the present invention provides hair dyeing which has good uptake, good selectivity, as well as good stability to light and shampooing with improved brilliance.

The compounds of the formula (I) are partly known and described for example in DE 2 230 392 and WO 02/076418. They can be prepared according to methods described in these documents or according to other methods which are known to a person of ordinary skill in the art.

However, compounds of the formula (I) wherein Z is -CH₂NR⁶R⁷ are novel and are also an object of this application.
Accordingly, the present invention refers to compounds of the formula (Iaa) 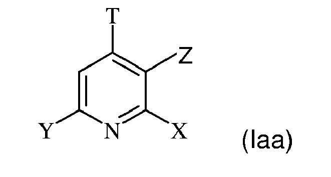 wherein
each of X and Y, independently, are -NR¹R², -NHSO₂R³, -NHC(O)R⁴or -OR⁵;
Z is -CH₂NR⁶R⁷;
T is alkyl, -OR²², -SR²³ or -C(O)OR²⁴;
each of R¹ to R⁵, independently, are alkyl, hydroxyalkyl, polyhydroxylalkyl, arylalkyl, alkoxyalkyl, thioalkoxyalkyl, poly(oxyalkylene)alkyl, aminoalkyl, N-monoalkyl-aminoalkyl, N-monoaryl-aminoalkyl, N,N-dialkyl-aminoalkyl, N,N-diaryl-aminoalkyl, N-alkyl-N-aryl-aminoalkyl, amino-hydroxy-alkyl, alkoxyalkylaminoalkyl, thioalkoxyalkyl-aminoalkyl, aminoalkyloxyalkyl, N-monoalkylamino-alkyloxyalkyl, N,N-dialkyl-aminoalkoxyalkyl, N-arylamino-alkoxyalkyl, N,N-diarylaminoalkoxyalkyl, N-alkyl-N-aryl-amino-alkoxyalkyl, aminoalkylthioxyalkyl, N-monoalkylamino-alkylthioxy-alkyl, N,N-dialkylamino-alkylthioxyalkyl, N-arylamino-alkylthioxyalkyl, N,N-diarylaminoalkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl; and
R¹, R² and R⁵ can additionally be hydrogen; or
R¹ and R² form together with the nitrogen to which they are bonded a 5- or 6-membered heterocyclic ring;
each of R⁶ and R⁷, independently are hydrogen, alkyl, hydroxyalkyl, polyhydroxylalkyl, arylalkyl, alkoxyalkyl, thioalkoxyalkyl, poly(oxyalkylene)alkyl, aminoalkyl, N-monoalkyl-aminoalkyl, N-monoaryl-aminoalkyl, N,N-dialkyl-aminoalkyl, N,N-diaryl-aminoalkyl, N-alkyl-N-aryl-aminoalkyl, amino-hydroxy-alkyl, alkoxyalkylaminoalkyl, thioalkoxyalkyl-aminoalkyl, aminoalkyloxyalkyl, N-monoalkylamino-alkoxyalkyl, N,N-dialkylamino-alkoxyalkyl, N-arylamino-alkoxyalkyl, N,N-diarylaminoalkoxyalkyl, N-alkyl-N-aryl-amino-alkoxyalkyl, aminoalkylthioxyalkyl, N-monoalkylamino-alkylthioxyalkyl, N,N-dialkylaminoalkylthioxyalkyl, N-arylamino-alkylthioxyalkyl, N,N-diarylamino-alkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl; and
each of R²² to R²⁴, independently are hydrogen, alkyl, hydroxyalkyl, polyhydroxylalkyl, arylalkyl, alkoxyalkyl, thioalkoalkyl, poly(oxyalkylene)alkyl, aminoalkyl, N-monoalkyl-aminoalkyl, N-monoaryl-aminoalkyl, N,N-dialkyl-aminoalkyl, N,N-diaryl-aminoalkyl, N-alkyl-N-aryl-aminoalkyl, amino-hydroxy-alkyl, alkoxyalkylaminoalkyl, thioalkoxyalkylaminoalkyl, aminoalkyloxyalkyl, N-monoalkylamino-alkyloxyalkyl, N,N-dialkylaminoalkyloxyalkyl, N-arylamino-alkyloxyalkyl, N,N-diarylaminoalkyloxyalkyl, N-alkyl-N-aryl-amino-alkyloxyalkyl, aminoalkylthioxyalkyl, N-monoalkylamino-alkyl-thioxyalkyl, N,N-dialkylaminoalkyl-thioxyalkyl, N-arylamino-alkylthioxyalkyl, N,N-diarylaminoalkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl.

As to alkyl, alkoxy, cycloalkyl and aryl groups and as to 5- or 6- membered heterocyclic rings, the disclosure given above for compounds of the formula (I) apply for compounds of formula (laa) as well.

In preferred compounds of the formula (laa) each of R⁶ and R⁷, independently is hydrogen or (C₁-C₄)-alkyl.
In especially preferred compounds of the formula (Iaa) Z is aminomethyl.

Also, in preferred compounds of the formula (Iaa) T is (C₁-C₆)-alkyl, -OR²² or -C(O)OR²⁴, wherein each of R²² and R²⁴, independently are hydrogen or (C₁-C₆)-alkyl.
In especially preferred compounds of the formula (Iaa) T is methyl, ethyl, hydroxy, - COOCH₃ or -COOC₂H₅.

Further, in preferred compounds of the formula (Iaa) each of X and Y, independently is
-NR¹R², wherein each of R¹ and R², independently is hydrogen, (C₁-C₆)-alkyl, hydroxy-(Cₗ-C₆)-alkyl, amino-(C₁-C₆)-alkyl, N-mono-(C₁-C₆)-alkyl-amino-(C₁-C₆)-alkyl or N,N-di-(C₁-C₆)-alkyl-amino-(C₁-C₆)-alkyl; or
-OR⁵, wherein R⁵ is hydrogen or (C₁-C₆)-alkyl.

In especially preferred compounds of the formula (laa) each of X and Y, independently is -NR¹R², wherein R¹ is hydrogen, methyl or ethyl; and R² is hydrogen, methyl, ethyl, hydroxymethyl, hydroxyethyl, dimethylaminomethyl, diethylaminomethyl, dimethylaminoethyl or diethylaminoethyl; or
X and Y both are -OH.

Examples of compounds of the formula (Iaa) are the compounds of formulae (Id), (Ie), (Ig), (Ih) and (In) given above.

The compounds of the formula (Iaa) can be prepared by reduction of the compounds of the formula (II) 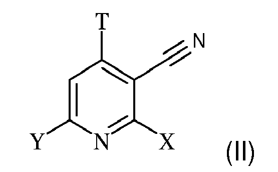 wherein X, Y and T are defined as given above to the compound of formula (III) 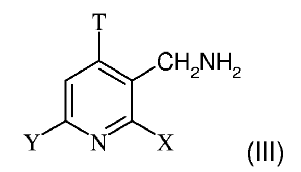 and optionally subsequent introduction of R⁶ and R⁷.

For example, compounds of the formula (Iaa) wherein Z is -CH₂NH₂ are obtained by reacting elemental hydrogen with the compound of the formula (lla) 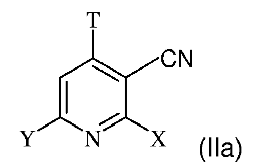 wherein X, Y and T are defined as given above, in the presence of palladium 5% on activated carbon powder as the most suitable hydrogenation catalyst. The catalyst is added to the reaction preferably in a ratio between 0.1 und 0.9 relating to the amount of the compound of the formula (IIa). It is further advantageous to conduct the catalytic hydrogenation in alcoholic-aqueous acid solutions at a pH < 1 and at a temperature ranging from room temperature to about 50 °C under a hydrogen pressure of preferably 20 bar.

Preferred embodiments of the invention are described in detail for the purpose of illustrating only and not for a limiting purpose.

### Example 1

### Preparation of (3-aminomethyl-4,N²,N⁶-trimethyl-pyridine-2,6-diamine dihydrochloride)

A mixture of 26.4 parts of 4-methyl-2,6-bis-methylamino-nicotinonitrile, 70 parts of concentrated hydrochloric acid and 250 parts of ethanol was hydrogenated over 14 parts of palladium 5% on activated carbon powder at a pressure of 20 bar and at 40°C for 72 hours. The catalyst was filtered off and the suction filter was washed several times with water. The filtrate was filtered trough Celite and distilled off. After drying in vacuum, the isolated product was finely ground to give 33.6 parts of a yellowish powder. The analytical data are consistent with the assigned structure for the compound of formula (Ig)

### Example 2

### Preparation of (2-[5-aminomethyl-6-(2-hydroxy-ethylamino)-4-methyl-pyridin-2-ylamino]-ethanol dihydrochloride)

A solution of 23.6 parts of 2,6-bis-(2-hydroxy-ethylamino)-4-methyl-nicotinonitrile in 80 parts of concentrated hydrochloric acid and 200 parts of ethanol was hydrogenated over 9 parts of palladium 5% on activated carbon powder at a pressure of 20 bar and at room temperature for 48 hours. The catalyst was filtered off and the suction filter was washed several times with water and ethanol. The filtrate was distilled off and after drying in vacuum the isolated product was finely ground to give 28.8 parts of a greenish yellow powder. The analytical data are consistent with the assigned structure for the compound of formula (In).

### Example 3

### Preparation of (2,6-dihydroxy-4-methyl-nicotinamide) 17.2 parts of 2,6-dihydroxy-4-methyl-nicotinonitrile were introduced while stirring and

external cooling into 100 parts of 85 % (by weight) sulfuric acid. Thereafter the cooling source was removed and the temperature was gradually increased up to 90°C. After 4 hours the conversion was complete. The reaction mixture was poured into 500 parts of cold water. The precipitate was filtered off and washed with water. Upon drying 18.6 parts of a brown powder were obtained. The analytical data are consistent with the assigned structure for the compound of formula (Ip).

### Example 4

### Preparation of (4-methyl-2,6-bis-methylamino-nicotinamide)

A mixture of 20.6 parts of 2,6-dichloro-4-methyl-nicotinonitrile and 75 parts of aqueous methylamine solution 40 wt. % in 250 parts of 2-propanol was agitated in an autoclave at 180 °C for 20 hours. Then the reaction mixture was distilled off and the residue stirred with 200 parts of water. The precipitate was filtered off, washed with water and dried to give 12.7 parts of a brown powder. The analytical data are consistent with the assigned structure for the compound of formula (Ik).

### Examples 5 to 23

Hair dye compositions according to the present invention were produced by mixing the following ingredients in amounts as given:

| | |
|---|---|
| Compound of the formula (I) | 0.8% |
| Developer a, b, c or d | 0.8% |
| lsopropanol | 10.0% |
| Ammonia 25% active | 10.0% |
| Sodium sulfite | 2.0% |
| Water qs. | 100.0 |

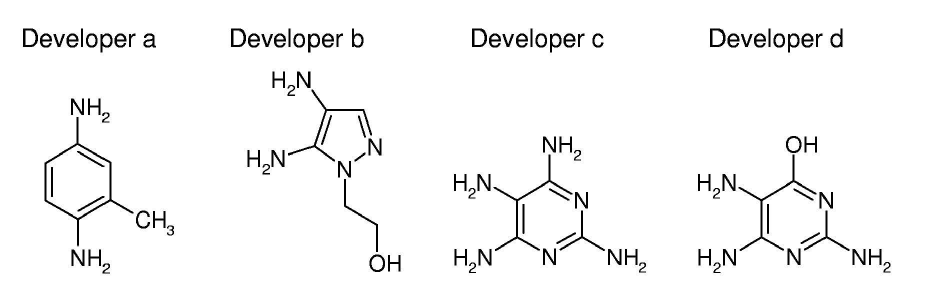

The hair dye compositions thus obtained are mixed in a 1:1 ratio with 6% aqueous peroxide and then applied to undamaged white goat hair at 50°C for 15 min. or 30°C for 30 min. Approximately, 1g of the mixtures of hair dye composition and peroxide are used per gram of hair. After the completion of the dyeing time, the tresses are rinsed with water, shampoo washed and then dried.
Brilliant colourings as given in the following table are obtained:

**Table 1**

| Example | Compound of the formula (I) | Developer a | Developer b |
|---|---|---|---|
| 5 | Id | brown | pink |
| 6 | Io | brown | red |
| 7 | mixture of In and It | dark brown | intense red |
| 8 | Ip | brown | intense orange-red |
| 9 | Ie | green | intense violet-brown |
| 10 | Iq | light brown | intense pink |
| 11 | If | mahogany | intense violet |
| 12 | Ig | brown | intense pink |
| 13 | Ih | light brown | violet |
| 14 | Ij | green | pink |
| 15 | Ik | intense mahogany | intense violet |
| 16 | Im | intense mahogany | orange |
| 17 | In | brown | violet |
| 18 | mixture of Ir and Is | intense violet brown | intense red |

**Table 2**

| Example | Compound of the formula (I) | Developer c | Developer d |
|---|---|---|---|
| 19 | Ih | intense yellow | intense orange |
| 20 | In | intense yellow | intense orange-yellow |
| 21 | mixture of Iu and It | yellow | light yellow |
| 22 | Ig | bright yellow | orange-yellow |
| 23 | lm | yellow | light yellow |

Table 3 shows the lightfastness of dyeings obtained with inventive compositions compared to dyeings obtained with compositions containing 2,6-diaminopyridine as coupler.

**Table 3**

| Pyridine Coupler | Developer | Dyeability Delta E | Lightfade Delta E |
|---|---|---|---|
| 2,6-diaminopyridine | a | 64 | 12 |
| 2,6-diaminopyridine | b | 72 | 11 |
| mixture of Iu and It | a | 53 | 5 |
| mixture of Iu and It | b | 58 | 8 |
| Ie | a | 43 | 9 |
| Im | b | 47 | 10 |

## Claims

1. Hair dye composition comprising at least one compound of formula (I) 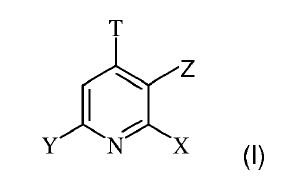 wherein
each of X and Y, independently, are -NR¹ R², -NHSO₂R³, -NHC(O)R⁴or -OR⁵;
Z is alkyl, -CH₂NR⁶R⁷, -CH₂N(SO₂R⁸)R⁹, -CH₂N(C(O)R¹⁰)R¹¹, -CH₂OR¹², -C(O)NR¹³R¹⁴, -C(S)NR¹⁵R¹⁶, -C(O)OR¹⁷, -CH=NR¹⁸, -C(alkoxy)=NR¹⁹, -C(O)R²⁰ or - C(S)R²¹;
T is alkyl, -OR²², -SR²³, or -C(O)OR²⁴;
each of R¹ to R⁵, independently, are alkyl, hydroxyalkyl, polyhydroxylalkyl, arylalkyl, alkoxyalkyl, thioalkoxyalkyl, poly(oxyalkylene)alkyl, aminoalkyl, N-monoalkyl-aminoalkyl, N-monoaryl-aminoalkyl, N,N-dialkyl-aminoalkyl, N,N-diaryl-aminoalkyl, N-alkyl-N-aryl-aminoalkyl, amino-hydroxy-alkyl, alkoxyalkylaminoalkyl, thioalkoxyalkyl-aminoalkyl, aminoalkyloxyalkyl, N-monoalkylamino-alkyloxyalkyl, N,N-dialkyl-aminoalkoxyalkyl, N-arylamino-alkoxyalkyl, N,N-diarylaminoalkoxyalkyl, N-alkyl-N-aryl-amino-alkoxyalkyl, aminoalkylthioxyalkyl, N-monoalkylamino-alkylthioxy-alkyl, N,N-dialkylamino-alkylthioxyalkyl, N-arylamino-alkylthioxyalkyl, N,N-diarylaminoalkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl; and
R¹, R²and R⁵ can additionally be hydrogen; or
R¹ and R² form together with the nitrogen to which they are bonded a 5- or 6-membered heterocyclic ring; and
each of R⁶to R²¹, independently are, alkyl, hydroxyalkyl, polyhydroxylalkyl, arylalkyl, alkoxyalkyl, thioalkoxyalkyl, poly(oxyalkylene)alkyl, aminoalkyl, N-monoalkyl-aminoalkyl, N-monoaryl-aminoalkyl, N;N-dialkyl-aminoalkyl, N;N-diaryl-aminoalkyl, N-alkyl-N-aryl-aminoalkyl, amino-hydroxy-alkyl, alkoxyalkylaminoalkyl, thioalkoxyalkyl-aminoalkyl, aminoalkyloxyalkyl, N-monoalkylamino-alkoxyalkyl, N,N-dialkylamino-alkoxyalkyl, N-arylamino-alkoxyalkyl, N,N-diarylaminoalkoxyalkyl, N-alkyl-N-aryl-amino-alkoxyalkyl, aminoalkylthioxyalkyl, N-monoalkylamino-alkylthioxyalkyl, N,N-dialkylaminoalkylthioxyalkyl, N-arylamino-alkylthioxyalkyl, N,N-diarylamino-alkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl; and
R⁶, R⁷, R⁹ and R¹¹ to R²⁰ can additionally be hydrogen;
each of R²² to R²⁴, independently are hydrogen, alkyl, hydroxyalkyl, polyhydroxylalkyl, arylalkyl, alkoxyalkyl, thioalkoalkyl, poly(oxyalkylene)alkyl, aminoalkyl, N-monoalkyl-aminoalkyl, N-monoaryl-aminoalkyl, N,N-dialkyl-aminoalkyl, N,N-diaryl-aminoalkyl, N-alkyl-N-aryl-aminoalkyl, amino-hydroxy-alkyl, alkoxyalkylaminoalkyl, thioalkoxyalkylaminoalkyl, aminoalkyloxyalkyl, N-monoalkylamino-alkyloxyalkyl, N,N-dialkylaminoalkyloxyalkyl, N-arylamino-alkyloxyalkyl, N,N-diarylaminoalkyloxyalkyl, N-alkyl-N-aryl-amino-alkyloxyalkyl, aminoalkylthioxyalkyl, N-monoalkylamino-alkyl-thioxyalkyl, N,N-dialkylaminoalkyl-thioxyalkyl, N-arylamino-alkylthioxyalkyl, N,N-diarylaminoalkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl; whereby
Z and T cannot be both alkyl;
when X and Y both are -OR⁵ and R⁵ is hydrogen, then Z cannot be alkyl or -CH₂OR¹² with R¹² being hydrogen; and
when Z is -C(O)NR¹³R¹⁴ with R¹³ and R¹⁴ both being hydrogen or one of R¹³ and R¹⁴ being hydrogen and the other methyl, then X and Y cannot both be -NR¹R² with R¹ and R² being hydrogen.

2. Hair dye composition according to claim1, wherein compound (I) is of the formula (Ia) 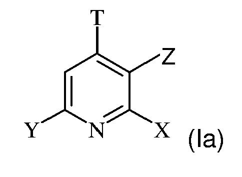 wherein
each of X and Y, independently, are -NR¹R²;
Z is -CH₂NR⁶R⁷, -CH₂N(SO₂R⁸)R⁹, -CH₂N(C(O)R¹⁰)R¹¹, -CH₂OR¹², -C(O)NR¹³R¹⁴or -C(S)NR¹⁵R¹⁶;
T is (C₁-C₆)-alkyl;
each of R¹, R² and R⁶to R¹⁶, independently, are (C₁-C₆)-alkyl, hydroxy-(C₁-C₆)-alkyl, amino-(C₁-C₆)-alkyl, N-mono-(C₁-C₆)-alkyl-amino-(C₁-C₆)-alkyl or N,N-di-(C₁-C₆)-alkyl-amino-(C₁-C₆)-alkyl and
R¹, R², R⁶, R⁷, R⁹ and R¹¹ to R¹⁶ can additionally be hydrogen,
whereby the compounds wherein Z is -CONH₂ or -CONHCH₃ and X and Y are both - NH₂ are excluded.

3. Hair dye composition according to claim1, wherein compound (I) is of the formula (Ib) 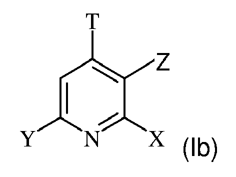 wherein
each of X and Y, independently, are -OR⁵;
Z is -C(O)NR¹³R¹⁴ or -C(O)OR¹⁷;
T is (C₁-C₆)-alkyl, -OR²² or -C(O)OR²⁴; and
each of R⁵, R¹³, R¹⁴, R¹⁷, R²² and R²⁴, independently, are hydrogen or (C₁-C₆)-alkyl.

4. Hair dye composition according to claim1, wherein compound (I) is of the formula (Ic) 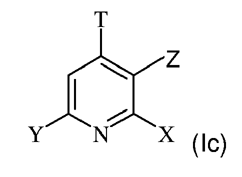 wherein
one of X and Y is -NR¹ R² and the other is -OR⁵;
Z is -C(O)NR¹³R¹⁴;
T is (C₁-C₆)-alkyl; and
each of R¹, R², R⁵, R¹³ and R¹⁴, independently, are hydrogen or (C₁-C₆)-alkyl.

5. Hair dye composition according to one or more of claims 1 to 4, which comprises in addition to the compounds of the formula (I) as couplers one or more developers, which couplers and developers are capable to form an oxidation dye for hair dyeing.

6. Hair dye composition according to one or more of claims 1 to 5, which comprises in addition to the compounds of the formula (I) further couplers.

7. Hair dye composition according to one or more of claims 1 to 6, which comprises at least one direct dye.

8. Hair dyeing kit which comprises in separate containers a hair dye composition according to one or more of claims 1 to 7, a developer composition and optionally further components.

9. Compound of the formula (laa) 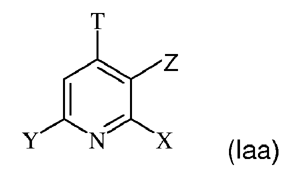 wherein
each of X and Y, independently, are -NR¹ R², -NHSO₂R³, -NHC(O)R⁴ or -OR⁵;
Z is -CH₂NR⁶R⁷;
T is alkyl, -OR²², -SR²³ or -C(O)OR²⁴;
each of R¹ to R⁵, independently, are alkyl, hydroxyalkyl, polyhydroxylalkyl, arylalkyl, alkoxyalkyl, thioalkoxyalkyl, poly(oxyalkylene)alkyl, aminoalkyl, N-monoalkyl-aminoalkyl, N-monoaryl-aminoalkyl, N,N-dialkyl-aminoalkyl, N,N-diaryl-aminoalkyl, N-alkyl-N-aryl-aminoalkyl, amino-hydroxy-alkyl, alkoxyalkylaminoalkyl, thioalkoxyalkyl-aminoalkyl, aminoalkyloxyalkyl, N-monoalkylamino-alkyloxyalkyl, N,N-dialkyl-aminoalkoxyalkyl, N-arylamino-alkoxyalkyl, N,N-diarylaminoalkoxyalkyl, N-alkyl-N-aryl-amino-alkoxyalkyl, aminoalkylthioxyalkyl, N-monoalkylamino-alkylthioxy-alkyl, N,N-dialkylamino-alkylthioxyalkyl, N-arylamino-alkylthioxyalkyl, N,N-diarylaminoalkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl; and
R¹, R²and R⁵ can additionally be hydrogen; or
R¹ and R² form together with the nitrogen to which they are bonded a 5- or 6-membered heterocyclic ring;
each of R⁶ and R⁷, independently are hydrogen, alkyl, hydroxyalkyl, polyhydroxylalkyl, arylalkyl, alkoxyalkyl, thioalkoxyalkyl, poly(oxyalkylene)alkyl, aminoalkyl, N-monoalkyl-aminoalkyl, N-monoaryl-aminoalkyl, N,N-dialkyl-aminoalkyl, N,N-diaryl-aminoalkyl, N-alkyl-N-aryl-aminoalkyl, amino-hydroxy-alkyl, alkoxyalkylaminoalkyl, thioalkoxyalkyl-aminoalkyl, aminoalkyloxyalkyl, N-monoalkylamino-alkoxyalkyl, N,N-dialkylamino-alkoxyalkyl, N-arylamino-alkoxyalkyl, N,N-diarylaminoalkoxyalkyl, N-alkyl-N-aryl-amino-alkoxyalkyl, aminoalkylthioxyalkyl, N-monoalkylamino-alkylthioxyalkyl, N,N-dialkylaminoalkylthioxyalkyl, N-arylamino-alkylthioxyalkyl, N,N-diarylamino-alkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl; and
each of R²² to R²⁴, independently are hydrogen, alkyl, hydroxyalkyl, polyhydroxylalkyl, arylalkyl, alkoxyalkyl, thioalkoalkyl, poly(oxyalkylene)alkyl, aminoalkyl, N-monoalkyl-aminoalkyl, N-monoaryl-aminoalkyl, N,N-dialkyl-aminoalkyl, N,N-diaryl-aminoalkyl, N-alkyl-N-aryl-aminoalkyl, amino-hydroxy-alkyl, alkoxyalkylaminoalkyl, thioalkoxyalkylaminoalkyl, aminoalkyloxyalkyl, N-monoalkylamino-alkyloxyalkyl, N,N-dialkylaminoalkyloxyalkyl, N-arylamino-alkyloxyalkyl, N,N-diarylaminoalkyloxyalkyl, N-alkyl-N-aryl-amino-alkyloxyalkyl, aminoalkylthioxyalkyl, N-monoalkylamino-alkyl-thioxyalkyl, N,N-dialkylaminoalkyl-thioxyalkyl, N-arylamino-alkylthioxyalkyl, N,N-diarylaminoalkylthioxyalkyl, N-alkyl-N-aryl-amino-alkylthioxyalkyl, cycloalkyl, cycloalkylalkyl, aryl, aryloxyalkyl, arylthioxyalkyl, heteroaryl, heteroarylalkyl, heterocycloalkyl or heterocycloalkylalkyl.

10. Compound according to claim 9, wherein each of R⁶ and R⁷, independently is hydrogen or (C₁-C₄)-alkyl.

11. Compound according to claim 9 and/or 10, wherein T is (C₁-C₆)-alkyl, -OR²² or - C(O)OR²⁴, wherein each of R²² and R²⁴, independently are hydrogen or (C₁-C₆)-alkyl.

12. Compound according to one or more of claims 9 to 11, wherein each of X and Y, independently is -NR¹R², wherein each of R¹ and R², independently is hydrogen, (C₁-C₆)-alkyl, hydroxy-(C₁-C₆)-alkyl, amino-(C₁-C₆)-alkyl, N-mono-(C₁-C₆)-alkyl-amino-(C₁-C₆)-alkyl or N,N-di-(C₁-C₆)-alkyl-amino-(C₁-C₆)-alkyl; or
-OR⁵, wherein R⁵ is hydrogen or (C₁-C₆)-alkyl.

13. Process for the preparation of a compound according to claim 1, by reduction of a compound of the formula (II) 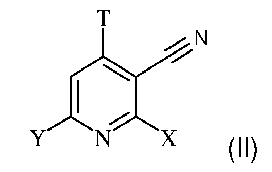 wherein X, Y and T are defined as given in claim 1 to the compound of formula (III) 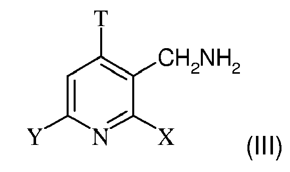 and optionally subsequent introduction of R⁶ and R⁷
